# EUROPEAN PATENT APPLICATION

(11) **EP 1 943 993 A2**
(43) Date of publication of application: **16.07.2008**
(21) Application number: 07255007.2
(22) Date of filing: 21.12.2007
(51) Int. Cl.: A61F 13/15

(54) **Package to accomodate feminine hygiene article**

(30) Priority: 22.12.2006 US 615306
(71) Applicant: Mcneil-PPC, Inc, Skillman, NJ 08558 (US)
(72) Inventor: Thornton,Kelly, Bensalem PA 19020 (US)
(74) Representative: Kirsch, Susan Edith

(57) **Abstract**

A package has plurality of pouches that are separable by the line of weakness, one pouch contains unused feminine hygiene article and other pouch is capable of receiving used feminine hygiene article.

## Description

### Field Of The Invention

The present invention generally relates to a packaged absorbent article and/or feminine hygiene article. The package includes a plurality of pouches, one of which contains the article, and a second is capable of receiving a used article.

### Background of Invention

Generally, women experience difficulty in deciding how to dispose of used feminine absorbent article, in particular used tampons. In general, there are two methods. One involves the disposal of the used tampon in the toilet. The other solution to disposal of a used tampon involves wrapping the used article, usually in toilet paper and placing the wrapped article in the trash. Both methods can be problematic and may result in plumbing problems or the presence of odor in the trash.

There have proposed solutions for used tampon disposal. See, for example, U.S. Pat. Nos. 6610037, 6393614, 6,687,911 and U.S. Pat. Pub. US20060212015. These proposed solution require the user to carry separate article, either a disposal bag, pouch or glove, in addition to the tampon. There are some packages or protective wrap, which initially package the feminine absorbent article. After the opening and removal of the unused absorbent article, the empty package can form a disposal receptacle for disposal of used feminine absorbent article. But since the used article must be disposed of prior to use of the unused article, the user will often have difficulty actually using the package. Also there are some packages that encompass the new tampon inside a disposal pouch. But in practice these disposing process of used feminine absorbent article founds to be tedious, time consuming and non-hygienic.

The present invention provides a single package that allows for the packaging of an absorbent article, such as a tampon, and also provides for a convenient way to dispose of the used article without requiring a separate disposal pouch. This invention provides a way to deliver an unused absorbent article and an easy disposal method of used feminine hygiene article.

### Summary of the Invention

According to a first aspect of the invention, the present invention provides a package adapted to accommodate a feminine hygiene article having a plurality of pouches, wherein a first pouch contains unused feminine hygiene article and a second pouch is capable of receiving used feminine hygiene article, pouches are separable by the line of weakness.

### Brief Description of the Drawings

Fig. 1 is a perspective view of the package assembly prior to opening;
Fig. 2 is a partial view of the opened package of Fig. 1;
Fig. 3 is a view of the packages of Fig.1 showing the package in an unrolled state; and
Fig.4 is a plan view of a pouch, which is capable to accommodate, used feminine absorbent article.

### Detailed Description of the Invention

The present invention generally relates to a packaged absorbent article and/or non-absorbent feminine hygiene article. The package includes a plurality of pouches, one of which contains the article, and a second is capable of receiving a used article.

As used herein, the term "absorbent article" may include diapers, sanitary napkins, pantiliners, tampons, interlabial devices or incontinence protectors and similar products. In particular, the feminine absorbent article may include digital tampons or applicator tampons.

As used herein, the term "non-absorbent feminine hygiene article" may include pessaries, supports, pads or patches. Again, this article may optionally be packaged with an applicator.

The second pouch is capable of receiving a used article, and it is separably attached to the first pouch in a manner that permits receiving such used article prior to removing the unused article from the first pouch. The second pouch may be separated from the first along a line of weakness at the border between the two pouches.

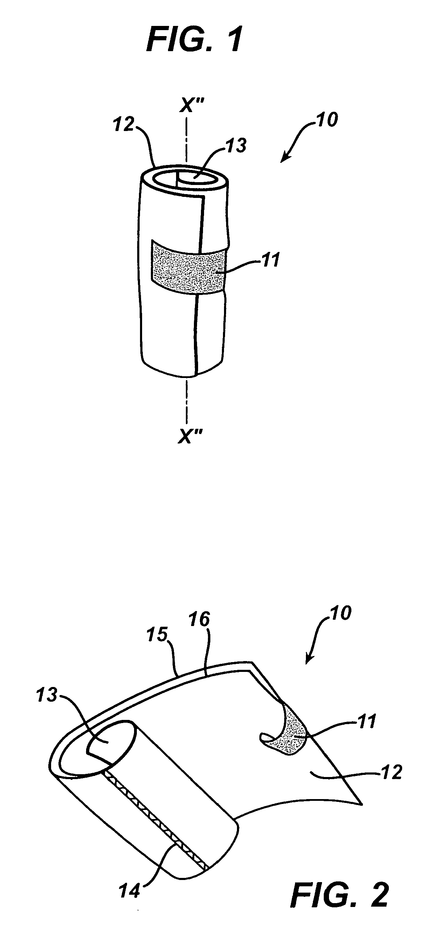

Referring to Fig. 1, which is an illustration of a package of the present invention in an unused configuration. The package however can be in many other forms, and is not limited to a structure having the particular configuration as shown in the drawings. As shown in this figure, package 10 has a first pouch 13 and a second pouch 12 and is convolutedly wound along axis X'X", which is how the package may appear prior to use. In this embodiment, adhesive tape 11 is used to secure the end of the second pouch 12 such that the package remains in a rolled form.

Both the pouches are separable by a line of weakness (14), as shown in FIG. 2. The line of weakness may be a continuous line of weakness, so that the second pouch to be detached for disposal can be completely detached and disposed of directly. It is also possible, however, to provide a line of weakness that is not completely continuous. Thus, although packaging of the used tampon is possible after destroying the line of weakness, the packaged used tampon continues to be connected to the packaged unused tampon. Therefore, after removal of the new tampon it is possible to dispose of the package in one piece.

The line of weakness is preferably a line with a reduced material thickness. As a result, On the one hand adequate stability of the package is ensured, which prevents inadvertent destruction of the line of weakness, on other hand intentional, defined destruction of the line of weakness is permitted. In addition, there are no openings in the material, so that the most stringent hygiene requirements are satisfied.

In the case of another embodiment, a perforated line defines the line of weakness. Such a perforated line is simpler in terms of technical production aspects and consequently less costly than the line of weakness with reduced material thickness described above. In this embodiment, the perforation holes may be very small.

In one embodiment, package 10 has first edge 15 and second edge 16, which together define openings for the first pouch 13 and second pouch 12.

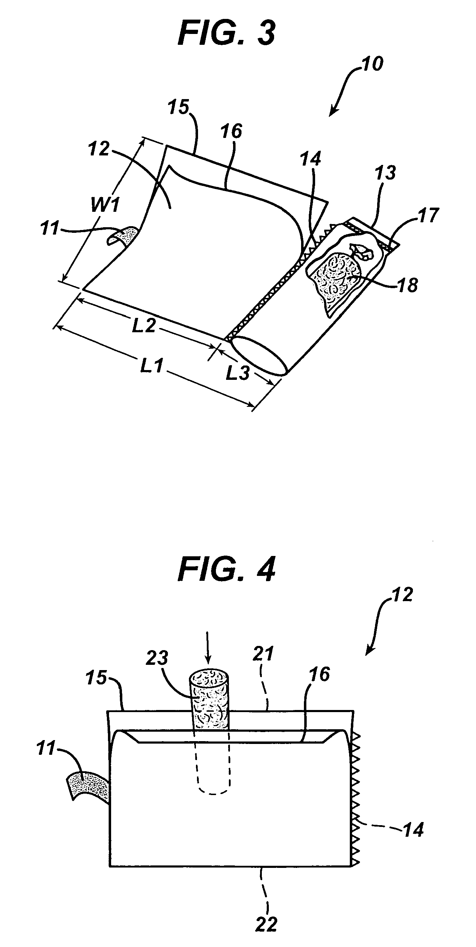

In one embodiment shown in Fig. 3 and 4, two pouches are separable by a line of weakness such as perforation (14), first pouch (13) which contains in this embodiment an unused tampon (18). Second pouch (12) is open at one end for receiving used feminine absorbent article, preferably digital tampon or applicator tampon formed by opening of first and second edges 15 and 16. In one embodiment, first edge 15 extends beyond second edge 16. One advantage to this type of configuration is easy opening. Another advantage to having the first edge 15 extend beyond second edge 16 is that this allows first edge 15 to form a flap and thereby fold over second edge 16. In one embodiment, first edge 15 extends beyond second edge 16 only in second pouch 13.

Package 10 has a length L1 and width W1, preferably L1 > W1, wherein first pouch (13) has Length L3 and width W1 and second pouch (12) is of length L2 and width W1, preferably L1>L2>L3. In one embodiment, second pouch 13 is used for disposal and first pouch 12 contains an unused tampon. In this embodiment, it is preferred that L2 be larger than L3. As used tampons and absorbent articles tend to swell upon absorbing fluid, the user may difficulty in placing the used product into the second pouch if the size is too small. Additionally, the user may be holding the pouch with one hand and trying to place the used article into the second pouch with her other hand. This may present some difficulties in just the handling aspects of the used product. In one embodiment, L2 of the second pouch is at least 1.5 times the size of L3 of the first pouch. In another embodiment, L2 of the second pouch is at least twice a large as the L3 of first pouch.

In this embodiment, line of weakness (14) between first pouch 13 and second pouch 12 can easily be broken by pulling the two pouches in opposite directions. In other embodiments, the line weakness can be broken by stretching until the pouches separate. Alternately, the line of weakness can include a tear strip.

In another embodiment, package 10 is sufficiently large enough to accommodate a tampon contained within an applicator. In another embodiment, package 10 is large enough to contain an external sanitary napkin or a pantiliner. Depending on the flexibility of the article used, package may not be tightly rolled about axis X'-X". Second pouch (12) is sufficient large enough to accommodate both a used tampon and applicator, and is larger than the first pouch (13).

In an advantageous embodiment, the second pouch is provided with a closure. As a result, the disposed-of tampon can, after packaging, be securely closed in its pouch, so that hygienic disposal is possible, even with other customary garbage, and significant leakage of fluid is reliably prevented. An adhesive closure, in particular a pressure-sensitive adhesive closure, may be provided as the closure. The part of the adhesive closure that is covered with an adhesive is in this case preferably provided with a pull-off covering, which is pulled off before sealing the used tampon within the pouch. However, it is also possible to insert into the second pouch material a drawstring with which second pouch can be closed.

Different adhesives, both adhesion adhesives and cohesion adhesives, may be used for the adhesive closure. The adhesives that can be used can be subdivided into physically setting adhesives and chemically setting adhesives.

The physically setting adhesives include hot-melt adhesives, such as SB (styrene-butadiene copolymers), EVA (ethylene-vinyl-acetate), polyesters; plastisol adhesives, such as for example PVC+plasticizer+coupling agent; pressure-sensitive adhesives, such as rubbers or polyacrylates; contact adhesives, such as PUR (polyurethane plastics) SB (styrene-butadiene copolymers); solvent/dispersion adhesives, such as VA (vinyl-acetate), VC (vinylchloride), VDC (vinylidene chloride) copolymers, EVA (ethylene-vinyl-acetate) or polyacrylates; or else glues based on starch, dextrin, PVAL (polyvinyl alcohol) or cellulose ether.

The chemically setting adhesives include, for example, reaction adhesives, such as for example EP (epoxy)+anhydrides, EP (epoxy)+polyamines, polyisocyanates+polyols, cyanoacrylates or UP (urethane polyester resins)+styrene or methacrylate.

In one embodiment, the package includes two pouches. In another embodiment, the package includes three pouches and may further include additional feminine hygiene articles such as a wipe. Additionally, the plurality of pouches may include a flexible pantyliner.

### Materials

The package of the present invention may be made from any materials that provide a sanitary package for delivering and disposing of an absorbent article. Preferred, material properties include without limitation, fluid impermeable, moisture resistant, flexible in nature, odor resistant, and biodegradable.

The package preferably comprises a plastic film, because this material is generally both impermeable to fluid and very inexpensive and at the same time has small packing dimensions and adequate stability, in order to avoid unwanted damage of the package.

For reasons of environmental protection, a degradable material may be used. Biodegradable materials with different degrading speeds may be used. A minimum degrading time is around 5 minutes, in order that the user has adequate time to place the used tampon in the second pouch and dispose of it before the degradable material noticeably breaks up.

Particularly preferred is a degrading time of one week to one month, whereby it is generally ensured that the tampon is disposed of in its state of being enclosed in the second pouch in a sealed manner by a disposal company within the regular time period. Suitable in particular as degradable materials are materials which have at least one polyester, in particular from the family of poly-.alpha.-hydroxyl acids, such as polytrimethylene carbonate, polydioxanone, poly-glycolide, polyactide, poly-(L-lactide-coglycolide) along with their copolymers with one another, polyorthoester and/or polycaprolactone, polyhydroxybutyrate or polyhydroxybutyrate-co-hydroxyvalerate. The polyhydroxybutyrate-co-hydroxyvalerate preferably has a valerate content of between 5 and 25%.

The degrading rate or degrading speed of the individual materials can be controlled by adding filler materials, for example starch, or by the choice of chain lengths or the number of branches.

In addition to these materials, a representative, non-limiting list of possible package material includes polymeric material such as EVA (ethylene-vinyl-acetate), EMA (ethylene-maleic anhydride copolymers), PE (polyethylene), PP (polypropylene), PET (polyester), PA (polyamide), PETP, PVA (polyvinyl alcohol), and the like; foils such as aluminium foil, aluminium oxide, silicon oxide, metalized sheets, and the like, an example of these latter three materials being Techbarrier S, V, H, T, AT, NR, NY Mitsubishi, Helional WTY (Amcor Flexibles), VA 535670 (metallised PE/PET) (Nordenia), 4364 (Schur-Flexible), and Coex HDPE Surlyn (Schur-Flexible).

An essentially opaque or colored material is preferably used, so that the disposal of the used tampon can take place discreetly and without being made visible.

## Claims

1. A hygienically packaged article comprising: a first pouch containing a hygienic article; and a second pound that is larger than the first pouch, separably attached to the first pouch, and capable of receiving a soiled article.

2. The packaged article according to claim 1, wherein the second pouch is separable from the first pouch along a line of weakness.

3. The packaged article according to claim 1, wherein the hygienic article comprises an absorbent article.

4. The packaged article according to claim 1, wherein the hygienic article comprises a feminine hygiene article.

5. The packaged article according to claim 1, wherein the second pouch is convolutedly wound over the first pouch.

6. The packaged article according to claim 1, wherein the second pouch comprises an opening sized and configured to accommodate a soiled article.

7. The packaged article according to claim 5, wherein the opening of the second pouch further comprises a closure.

8. The packaged article according to claim 7, wherein the closure of the second pouch comprises a re-sealable element.

9. The packaged article according to claim 1, wherein the hygienic article comprises a vaginal tampon.

10. The packaged article according to claim 1, wherein the second pouch further comprises an additive selected from the group consisting of fragrance, disinfectant, a protective coating to prevent leakage from the soiled article, and combinations thereof.

11. The packaged article according to claim 10, wherein the protective coating provides a hydrophobic barrier.

12. The packaged article according to claim 1, wherein at least one pouch comprises a polymeric film selected from the group consisting of polyethylene, polypropylene, polyester, cellophane, polyamide, poly(vinyl chloride), ethylene-vinyl acetate copolymer, polystyrene and polyethylene-based esters like polyethylene-terephthalate, heat shrinkable material, stretch foils, pre-stretched elastic material, cellulosic papers or metallic foils and combinations thereof.
